# EUROPEAN PATENT APPLICATION

(11) **EP 4 850 535 A1**
(43) Date of publication of application: **09.12.2026**
(21) Application number: 25748159.8
(22) Date of filing: 27.01.2025
(51) Int. Cl.: A23F 5/46

(54) **ENZYME AGENT, PROCESSED COFFEE AND APPLICATION THEREOF**

(30) Priority: 01.02.2024 CN 202410143724; 25.12.2024 CN 202411929641
(71) Applicant: Amano Enzyme Inc., Nagoya-shi Aichi 460-8630 (JP); Amano Enzyme Manufacturing (China), Ltd., Suqian City, Jiangsu Province, 223700 (CN)
(72) Inventor: GUO yuxian, Wuxi, Jiangsu 214122 (CN); HU jianli, Shanghai 200040 (CN); TORIYAMA Tomoe, Shanghai 200040 (CN); WANG Panhui, Wuxi, Jiangsu 214122 (CN)
(74) Representative: Witthoff Jaekel Steinecke Patentanwälte PartG mbB
(86) International application number: PCT/JP2025/002482
(87) International publication number: WO 2025/164577

(57) **Abstract**

It is an object of the present invention to provide a coffee beverage in which aggregation of coffee oil is suppressed. The present invention relates to an enzyme agent containing a lipase derived from Candida sp., which is for suppressing aggregation of coffee oil. In addition, the present invention relates to a processed coffee, in which an enzyme agent is allowed to act on coffee, and a method for producing a processed coffee, including allowing an enzyme agent to act on coffee.

## Description

### Technical Field

The present invention relates to an enzyme agent, a processed coffee, a method for producing a processed coffee, and a method for suppressing aggregation of coffee oil.

### Background Art

Coffee beverage consumption is increasing year by year, and in particular, the Chinese market is showing explosive growth due to rising national income and changing consumer consciousness. In a coffee beverage-producing process, a filtration treatment and sterilization treatment are performed on the extracted coffee.

In an extraction step or a filtration treatment step in the coffee beverage-producing process, addition of various enzymes has been considered to enhance the amount of components in the extract and to improve filterability. For example, it has been known that mannanase acts on galactomannan contained in coffee, and improves the extraction rate of coffee components and exhibits a precipitation-preventing effect. On the other hand, it has been known that cellulase acts on cellulose, the main component of the cell wall of coffee beans, and increases the solubility of the coffee beans, thereby improving the extraction rate and exhibiting a precipitation-preventing effect.

Moreover, in the coffee beverage-producing process, addition of enzymes to enhance the *umami* (deliciousness) and flavor of coffee has been considered. For example, Patent Document 1 discloses a method for producing a coffee flavor by treating coffee oil with lipase. Here, production of a coffee flavor product with a smooth texture, throat feel, and espresso-like taste is being considered by allowing lipase to act on coffee bean-derived oil.

### Prior Art Document

### Patent Document

Patent Document 1: JP Patent Publication No. 2007-61046 A

### Summary of Invention

### Objects to be Solved by the Invention

Since coffee beans contain oils and fats, in a process of preserving coffee extracted from coffee beans, the oils and fats continuously float to the surface of the coffee and aggregate (phase separation), so that an oil ring may be generated in some cases.

Thus, it is an object of the present invention to provide a coffee beverage in which aggregation of coffee oil is suppressed.

### Means for Solving the Objects

Examples of specific aspects of the present invention will be described below.
[1] An enzyme agent containing a lipase derived from Candida sp., which is for suppressing aggregation of coffee oil.
[2] A processed coffee, in which the enzyme agent according to [1] is allowed to act on coffee.
[3] The processed coffee according to [2], in which the moisture content of the coffee is 80% by mass or more.
[4] A method for producing a processed coffee, including allowing a lipase derived from Candida sp. to act on coffee.
[5] The method for producing a processed coffee according to [4], in which the moisture content of the coffee is 80% by mass or more.
[6] A method for suppressing aggregation of coffee oil, including allowing a lipase derived from Candida sp. to act on coffee.

### Advantageous Effects of Invention

According to the present invention, a coffee beverage in which aggregation of coffee oil is suppressed can be provided. In addition, according to the present invention, a coffee beverage with excellent preservation stability can be provided by suppressing aggregation of coffee oil.

### Embodiments of Carrying out the Invention

The present invention will be described in detail below. The explanation given below may be described based on some typical embodiments or specific examples. However, the present invention should not be limited to such embodiments. In the present description, the numerical range expressed with the wording "a number to another number" means the range that falls between the former number indicating the lower limit value of the range and the latter number indicating the upper limit value thereof.

### (Enzyme agent)

The present invention relates to an enzyme agent containing a lipase, which is for suppressing aggregation of coffee oil. Among others, a preferred embodiment of the present invention relates to an enzyme agent containing a lipase derived from Candida sp., which is for suppressing aggregation of coffee oil. In the present embodiment, aggregation of coffee oil can be suppressed by allowing a lipase derived from Candida sp. to act on coffee, thereby suppressing generation of oil rings. It is to be noted that, in the present description, an "oil ring" refers to a ring-shaped oil layer that is formed on the surface of the liquid in a container due to separation of coffee oil contained in the coffee during the storage through hydrophobic interactions.

The enzyme agent is not particularly limited, as long as it is an agent containing a lipase derived from Candida sp. The enzyme agent may also be an agent consisting of a lipase derived from Candida sp. Moreover, the enzyme agent may be in any form such as powders, a solid, a gel, or a liquid.

Furthermore, the enzyme agent may contain a carrier, and in this case, the enzyme within the enzyme agent may be immobilized on a carrier such as a porous body. In the case of immobilized enzymes, the enzyme and the carrier may be covalently bonded to each other, may be attracted to each other by electrostatic interactions, or the enzyme may be encapsulated in a protein within a porous body. The carrier used for immobilized enzymes is not particularly limited, and it is preferably insoluble. The insoluble carrier may be either an inorganic or organic material.

### < Lipase >

Lipase is an enzyme that catalyzes a reaction of acting on triacylglycerol (triglyceride) to allow the triacylglycerol (triglyceride) to release fatty acid depending on the specificity of the enzyme, so as to produce diacylglycerol (DAG) or monoacylglycerol (MAG), or a reverse reaction thereof.

Examples of the lipase may include a lipase derived from Penicillium sp., a lipase derived from Aspergillus sp., a lipase derived from Rhizopus sp., a lipase derived from Candida sp., a lipase derived from Mucor sp., a lipase derived from Rhizomucor sp., and a lipase derived from Penicillium sp. In the present embodiment, the lipase is preferably a lipase derived from Candida sp., and is particularly preferably a lipase derived from *Candida cylindracea*. In the present embodiment, by using such a lipase derived from Candida sp., generation of oil rings can be effectively suppressed. Furthermore, in the present embodiment, by using a lipase derived from Candida sp., generation of the off-flavors of coffee (for example, the deterioration odor of coffee) can also be suppressed.

Lipase can be prepared from a culture medium of microorganisms serving as an origin of lipase as mentioned above. A specific preparation method may be a method of recovering lipase from the culture medium or cell mass of the above-described microorganisms. For example, in the case of using lipase-secreting microorganisms, the cell mass has previously been recovered from the culture medium by filtration, centrifugation, etc., as necessary, and the enzyme is then separated and/or purified. In the case of using lipase-non-secreting microorganisms, the cell mass has previously been recovered from the culture medium, as necessary, and the recovered cell mass is then ground by a pressure treatment, an ultrasonic treatment, etc. to expose the enzyme, and the enzyme is then separated and/or purified. The method of separating and/or purifying the enzyme is not particularly limited, and a known protein separation and/or purification method can be adopted. Examples of such a protein separation and/or purification method may include a centrifugal separation method, a UF concentration method, a salting-out method, and various types of chromatographic methods using ion exchange resin, etc. The separated and/or purified enzyme can be powderized by drying methods such as freeze-drying and drying under reduced pressure. Otherwise, the separated and/or purified enzyme can also be powderized by using suitable excipients and/or drying aids in such drying methods. Moreover, the separated and/or purified enzyme can also be liquefied by adding suitable additives thereto and performing filter sterilization.

A commercially available product can also be used as such a lipase. An example of a preferred commercially available product may be a lipase derived from *Candida cylindracea*, which is manufactured by Amano Enzyme Inc.

When the enzyme agent is allowed to act on coffee, the additive amount of a lipase to be acted is preferably 0.001% by mass or more, more preferably 0.01% by mass or more, and further preferably 0.05% by mass or more, with respect to the total mass of coffee. On the other hand, the additive amount of a lipase to be acted is preferably 20% by mass or less, and more preferably 10% by mass or less, with respect to the total mass of coffee.

Moreover, although the additive amount of a lipase to be acted is not particularly limited, it is preferable to add the lipase, so that the enzyme activity becomes preferably 0.1 U or more, more preferably 0.5 U or more, even more preferably 1 U or more, further preferably 10 U or more, even further preferably 50 U or more, and still further preferably 100 U or more, with respect to 1 g of coffee. On the other hand, regarding the additive amount of the lipase, it is preferable add the lipase, so that the enzyme activity becomes preferably 50000 U or less, more preferably 30000 U or less, even more preferably 10000 U or less, further preferably 5000 U or less, even further preferably 2000 U or less, and still further preferably 1000 U or less, with respect to 1 g of coffee.

The enzyme activity of a lipase is measured by the following method. First, 18 g of polyvinyl alcohol I and 2 g of polyvinyl alcohol II are weighed out, 800 mL of water is added thereto, and while stirring, the resultant is heated at 75°C to 80°C for about 1 hour for dissolution. The solution obtained by diluting the above solution with water to 1000 mL is defined as a "solution." On the other hand, 150 mL of an emulsion and 50 mL of olive oil (Japanese Pharmacopoeia) are emulsified using a homogenizer under conditions of 10°C or lower to obtain an "olive oil emulsion." After the preparation, the olive oil emulsion is stored in a refrigerator and is left for 1 hour or more before use.

Next, 4 mL of McIlvaine buffer (pH 7.0) and 5 mL of the olive oil emulsion are mixed with each other, and the mixture is then left at rest at 37°C for 10 to 15 minutes. To this mixture, 1 mL of an enzyme solution diluted to a predetermined concentration is added, is mixed, and is left at 37°C for 30 minutes. Thereafter, 10 mL of an ethanol/acetone mixed solution (1 : 1) is added to and mixed with the reaction mixture, and to the thus obtained mixture, 10 mL of 0.05 mol/L sodium hydroxide and 10 mL of an ethanol/acetone mixed solution (1 : 1) were added, and further, a phenolphthalein reagent is added thereto as an indicator. While stirring with a stirrer under nitrogen replacement, 0.05 mol/L hydrochloric acid is titrated until the pH reaches 10.00, so as to determine T₃₀ mL. As a blank, one obtained by adding 10 mL of an ethanol/acetone mixed solution (1 : 1) to 4 mL of the buffer and 5 mL of the olive oil emulsion, mixing them, and adding 1 mL of water thereto, is used, and the same operations as those described above are then performed to determine T₀ mL. Regarding lipase activity, using olive oil (Japanese Pharmacopoeia) as a substrate, the amount of an enzyme that provides an increase of 1 micromol of fatty acid per minute when the olive oil is treated at 37°C is defined as 1 unit (1 U), and the lipase activity is calculated using the following equation. [Expression 1] $Lipase activity = 50 \times \left({T}_{0}-{T}_{30}\right) \times \frac{1}{30} \times f \times n = \frac{\left({T}_{0}-{T}_{30}\right) \times f \times n}{0.6}$

### < Coffee >

The coffee is coffee in a liquid or semi-liquid form. For example, the coffee may be an extract obtained by extracting coffee raw materials (raw coffee beans, roasted coffee beans, or ground coffee beans obtained by grinding the aforementioned coffee beans) with water (including hot water or steam), or a concentrate obtained by concentrating the extract, etc. It is to be noted that the concentrate may be a semi-liquid substance, such as a paste, obtained by concentrating the coffee. Moreover, the coffee may be an instant coffee obtained by dissolving a dried product (such as instant coffee powders, etc.) of the above-mentioned extract or concentrate in water (including hot water or steam). Furthermore, the coffee also includes a slurry in which coffee raw materials are dispersed, or a solution in which coffee raw materials have settled.

Among these, in the present embodiment, the coffee is preferably an extract of raw coffee beans, roasted coffee beans, or ground coffee beans obtained by grinding the aforementioned coffee beans. The type of coffee beans used for the coffee is not particularly limited, and examples of cultivated tree species may include, for example, Arabica, Robusta, and Liberica. Examples of the coffee variety may include Mocha, Brazil, Colombia, Guatemala, Blue Mountain, Kona, Mandheling, and Kilimanjaro. One type of coffee beans may be used, or a blend of multiple types of coffee beans may also be used. In the present embodiment, it is preferable to roast the coffee beans to make roasted coffee beans, and the coffee is preferably an extract of roasted coffee beans.

In the present embodiment, the coffee is preferably an extract obtained by extraction from roasted coffee beans or their ground materials using water (including hot water or steam). The coffee is preferably a water (including hot water or steam) extract, and the moisture content of the coffee is preferably 80% by mass or more, more preferably 85% by mass or more, even more preferably 90% by mass or more, further preferably 95% by mass or more, and particularly preferably 99% by mass or more.

The pH of the coffee is, for example, pH 3.0 to 7.0, preferably pH 3.5 to 6.5, more preferably pH 4.0 to 6.0, and further preferably pH 4.2 to 5.0.

In the present embodiment, the coffee is preferably coffee beans, or an extract of a ground product of the coffee beans, and the oil and fat content of the coffee beans is preferably 1.0% by mass or more, more preferably 1.2% by mass or more, further preferably 1.4% by mass or more, and particularly preferably 1.5% by mass or more.

### (Processed coffee)

The present embodiment relates to a processed coffee, in which the aforementioned enzyme agent is allowed to act on coffee. The processed coffee contains enzyme-treated products of some components of the coffee, and in the present embodiment, it is preferable that the processed coffee contains fatty acids.

In the present embodiment, the coffee on which the enzyme agent is allowed to act is preferably a water (including hot water or steam) extract. Thus, the moisture content of the processed coffee obtained is preferably 80% by mass or more, more preferably 85% by mass or more, even more preferably 90% by mass or more, further preferably 95% by mass or more, and particularly preferably 99% by mass or more.

The acid value of the processed coffee is preferably 1.10 mg KOH or more. On the other hand, the acid value of a processed coffee is preferably 10.0 mg KOH or less, more preferably 8.00 mg KOH or less, and further preferably 5.00 mg KOH or less. The acid value of a processed coffee can be calculated by neutralization titration using potassium hydroxide and a pH indicator.

It is to be noted that the processed coffee in the present description includes a slurry in which coffee raw materials are dispersed, a solution in which coffee raw materials have settled, and a dried solid processed coffee.

### (Processed coffee-containing beverage)

The present embodiment may relate to a processed coffee-containing beverage containing the aforementioned processed coffee. In the processed coffee-containing beverage of the present embodiment, optional components may be present, as appropriate. Examples of the optional components that may be added to the processed coffee-containing beverage may include milk (animal milk, plant milk), flavors, sweeteners, acidulants, bittering agents, coloring agents, antioxidants, pH adjusters, vitamins, amino acids, minerals, antifoaming agents, emulsifiers (glycerin fatty acid esters, sucrose fatty acid esters, lecithin, saponins, etc.), thickening polysaccharides (pectin, carboxymethyl cellulose, etc.), and salts (sodium chloride, calcium salts, phosphates, etc.).

For the purpose of further improving the effect of suppressing generation of oil rings, the processed coffee-containing beverage may use a pH adjuster. The pH adjuster is not limited, as long as it is a substance that exhibits alkalinity when it is dissolved in water. Specific examples of the pH adjuster may include sodium bicarbonate (sodium hydrogen carbonate), sodium carbonate, potassium carbonate, sodium hydroxide, potassium hydroxide, trisodium phosphate, and tripotassium phosphate. These pH adjusters may be used alone as a single type, or may also be used in combination of multiple types. Among these pH adjusters, sodium bicarbonate (sodium hydrogen carbonate) is preferable from the viewpoint of further improving the effect.

When a pH adjuster is added to the processed coffee-containing beverage, the amount of the pH adjuster used is not particularly limited, and for example, the amount of pH adjuster per 100 parts by mass of coffee is preferably 1 to 1000 parts by mass, more preferably 1 to 500 parts by mass, further preferably 1 to 100 parts by mass, and particularly preferably 1 to 50 parts by mass.

### (Method for producing processed coffee)

The present embodiment relates to a method for producing a processed coffee, which includes allowing a lipase derived from Candida sp. to act on coffee.

The reaction time, the temperature, and the pH of the reaction solution for allowing the enzyme agent to act on coffee are not particularly limited. The reaction temperature is, for example, 5°C to 75°C, preferably 10°C to 75°C, more preferably 15°C to 75°C, even more preferably 20°C to 60°C, further preferably 25°C to 55°C, still further preferably 30°C to 55°C, and particularly preferably 40°C to 55°C. The pH of the reaction solution is, for example, pH 3.0 to 10.0, preferably pH 3.0 to 7.0, and more preferably pH 4.0 to 7.0. The reaction time is, for example, 1 minute to 24 hours, preferably 2 minutes to 12 hours, and more preferably 10 minutes to 6 hours. By applying the aforementioned reaction conditions, generation of oil rings can be more effectively suppressed, and further, generation of the off-flavors of coffee (for example, the deterioration odor of coffee) can be more effectively suppressed. Moreover, in the present embodiment, even when the enzyme treatment is performed under a relatively low-temperature environment of 15°C or lower, generation of oil rings can be more effectively suppressed, and further, generation of the off-flavors of coffee (for example, the deterioration odor of coffee) can also be more effectively suppressed. It is to be noted that these reaction conditions are appropriately selected depending on the type of coffee used, etc. The optimal reaction conditions may be determined through preliminary experiments.

One aspect of the method for producing a processed coffee of the present embodiment includes the following steps (1) and (2). It is to be noted that a step of inactivating the enzyme may be added after the following step (2).
(1) A step of preparing coffee.
(2) A step of treating the prepared coffee with the enzyme agent containing a lipase derived from Candida sp.

By using the above-described production method, a processed coffee, in which generation of oil rings is suppressed, and further, generation of the off-flavors of coffee (for example, the deterioration odor of coffee) is suppressed, can be produced.

The coffee prepared in the above step (1) is preferably an extract obtained by extraction from roasted coffee beans or their ground materials, using water (including hot water or steam). Moreover, the moisture content of the coffee prepared in the step (1) is preferably 80% by mass or more, more preferably 85% by mass or more, even more preferably 90% by mass or more, further preferably 95% by mass or more, and particularly preferably 99% by mass or more.

### (Aggregation-suppressing method)

The present embodiment relates to a method for suppressing aggregation of coffee oil, including allowing a lipase derived from Candida sp. to act on coffee. In the aggregation-suppressing method of the present embodiment, by allowing the above-mentioned enzyme agent to act on coffee, aggregation of coffee oil can be suppressed, and generation of oil rings can be thereby suppressed.

### Examples

Hereinafter, the characteristics of the present invention will be more specifically described in the following examples and comparative examples. The materials, amounts used, proportions, treatment contents, treatment procedures, etc. shown in the following examples may be changed, as appropriate, without deviating from the gist of the present invention. Therefore, the scope of the present invention should not be interpreted limitedly by the specific examples shown below.

### (Materials used)

**[Table 1]**

| Raw material name | Shape | Manufacturer |
|---|---|---|
| Ground coffee beans | Large grains | UCC Ueshima Coffee (Shanghai) Co., Ltd. |
| Coffee liquid | Liquid | UCC Ueshima Coffee (Shanghai) Co., Ltd. |

**[Table 2]**

| Enzyme agent | Manufacturer |
|---|---|
| Lipase derived from *Candida cylindracea* | manufactured by Amano Enzyme Inc. |
| Lipase derived from *Rhizopus delemer* | manufactured by Amano Enzyme Inc. |
| Lipase (1) derived from *Rhizopus oryzae* | manufactured by Amano Enzyme Inc. |

### (Enzyme activity measurement method)

18 g of polyvinyl alcohol I and 2 g of polyvinyl alcohol II were weighed out, 800 mL of water was added thereto, and while stirring, the resultant was heated at 75°C to 80°C for about 1 hour for dissolution. The solution obtained by diluting the above solution with water to 1000 mL was defined as a "solution." On the other hand, 150 mL of an emulsion and 50 mL of olive oil (Japanese Pharmacopoeia) were emulsified using a homogenizer under conditions of 10°C or lower to obtain an "olive oil emulsion." After the preparation, the olive oil emulsion was stored in a refrigerator and was left for 1 hour or more before use.

4 mL of McIlvaine buffer (pH 7.0) and 5 mL of the olive oil emulsion were mixed with each other, and the mixture was then left at rest at 37°C for 10 to 15 minutes. To this mixture, 1 mL of an enzyme solution diluted to a predetermined concentration was added, was mixed, and was left at 37°C for 30 minutes. Thereafter, 10 mL of an ethanol/acetone mixed solution (1 : 1) was added to and mixed with the reaction mixture. To the thus obtained mixture, 10 mL of 0.05 mol/L sodium hydroxide and 10 mL of an ethanol/acetone mixed solution (1 : 1) were added, and further, a phenolphthalein reagent was added thereto as an indicator. While stirring with a stirrer under nitrogen replacement, 0.05 mol/L hydrochloric acid was titrated until the pH reached 10.00, and the amount titrated was defined as T₃₀ mL. As a blank, one obtained by adding 10 mL of an ethanol/acetone mixed solution (1 : 1) to 4 mL of the buffer and 5 mL of the olive oil emulsion, mixing them, and adding 1 mL of water thereto, was used, and 0.05 mol/L hydrochloric acid was titrated until the pH reaches 10.00. The amount titrated was defined as T₀ mL. Regarding lipase activity, using olive oil (Japanese Pharmacopoeia) as a substrate, the amount of an enzyme that provides an increase of 1 micromol of fatty acid per minute when the olive oil was treated at 37°C was defined as 1 unit (1 U), and the lipase activity was calculated using the following equation. [Expression 2] $Lipase activity = 50 \times \left({T}_{0}-{T}_{30}\right) \times \frac{1}{30} \times f \times n = \frac{\left({T}_{0}-{T}_{30}\right) \times f \times n}{0.6}$

In the above equation, f is the factor for 0.05 mol/L hydrochloric acid (for quantification), and n is the dilution magnification per 1 ml of sample.

### [Example 1]

### (Method for producing coffee)

A coffee machine was connected to a power source, and was preheated by pressing the heating button. Weighed ground coffee beans (9.0 g) were placed in a powder bowl, were leveled, and were set in the coffee maker. When the temperature of the preheated water increased to between 100°C and 104°C, the extraction button was pressed to begin extraction. The timer was started when a first drop of coffee (extract) flowed out, and the extraction button was pressed at 11 seconds to stop the water injection. Coffee continued to flow even after the extraction was terminated, and the recovery of the coffee liquid was terminated after 1 minute. The amount of the coffee recovered was 48.6 g. Hot water (26.1 g) was added, so that the Brix was adjusted to be 1.0%. In this way, 74.7 g of coffee was obtained. By repeating the above-described operations, a predetermined amount of coffee was obtained.

95 mL of coffee was dispensed into a reaction vessel. A lipase derived from *Candida cylindracea* was added to the coffee to 0.1 %W/V (503 U lipase activity per gram of the coffee). The reaction was carried out in a 30°C water bath for 50 minutes. After completion of the reaction, the enzyme was inactivated by being treated in a boiling water bath for 10 minutes, and a sample for acid value measurement was then collected. The processed coffee obtained after the reaction was transferred to a 100 mL glass vial, and stored under refrigeration at 5°C, and the presence or absence of generation of oil rings (aggregates of coffee oil) was observed.

### [Comparative Example 1]

Coffee (control) was produced in the same manner as that of Example 1, with the exception that a lipase derived from *Candida cylindracea* was not added, and the coffee was produced in the same manner as that of Example 1.

### [Comparative Examples 2 and 3]

Processed coffee was obtained in the same manner as that of Example 1, with the exception that a lipase derived from *Rhizopus delemer* (Comparative Example 2) or a lipase (1) derived from *Rhizopus oryzae* (Comparative Example 3) was added, instead of the lipase derived from *Candida cylindracea*.

### [Evaluation 1] Oil ring formation

The coffee or the processed coffee obtained from each of the example and the comparative examples was stored at 5°C, and the presence or absence of oil ring formation was observed on Days 1, 7, 14, and 21. The results were evaluated using the following scale: "+++: Thick oil ring formed, ++: Oil ring formed, +: Slight oil ring formed, and -: No oil ring formed."

**[Table 3]**

| Observation of oil ring in processed coffee | | | | | |
|---|---|---|---|---|---|
| | | Store in refrigerator at 5°C | | | |
| | | Day 1 | Day 7 | Day 14 | Day 21 |
| Comp. Ex. 1 | Untreated with enzyme | ++ | +++ | ++ | +++ |
| Example 1 | Lipase derived from *Candida cylindracea* | - | - | - | + |
| Comp. Ex. 2 | Lipase derived from *Rhizopus delemer* | ++ | +++ | +++ | ++ |
| Comp. Ex. 3 | Lipase (1) derived from *Rhizopus oryzae* | ++ | +++ | ++ | ++ |

### [Evaluation 2] Calculation of relative acid value and sensory testing

The acid value of the coffee or the processed coffee obtained from each of the example and the comparative examples was calculated by neutralization titration using potassium hydroxide and a pH indicator. The acid value was calculated based on the weight (mg) of potassium hydroxide necessary for neutralizing the free fatty acids contained in 1 g of the sample. Moreover, the relative acid value of the processed coffee liquid was calculated while setting the acid value of a sample untreated with the enzyme as 100%, and it is shown in Table 4. In addition, after storing the processed coffee at 5°C for 14 days, evaluation of the off-flavors of the coffee was carried out with four panelists. A score of 3 points indicated "strongly perceived off-flavors of stale coffee (the deterioration odor of coffee)," 2 points indicated "perceived off-flavors of stale coffee," 1 point indicated "slightly perceived off-flavors of stale coffee," and 0 points indicated "no perceived off-flavors of stale coffee." The average scores are shown in Table 4 below.

**[Table 4]**

| Relative acid value immediately after enzyme treatment and sensory evaluation results of processed coffee stored at 5°C for 2 weeks | | | | |
|---|---|---|---|---|
| | | Acid value (mg·KOH) | Relative acid value (%) | Average score |
| Comp. Ex. 1 | Untreated with enzyme | 1.09 | 100 | 2.0 |
| Example 1 | Lipase derived from *Candida cylindracea* | 1.20 | 109.3 | 0.25 |
| Comp. Ex. 2 | Lipase derived from *Rhizopus delemer* | 1.15 | 104.7 | 0.75 |
| Comp. Ex. 3 | Lipase (1) derived from *Rhizopus oryzae* | 1.13 | 102.9 | 3.0 |

In the example, despite an increase in the relative acid value (indicating fatty acid generation) compared to Comparative Examples 2 and 3, the result was that no off-flavor was detected. In Comparative Example 3, despite a less significant increase in relative acid value compared to Comparative Example 2, that result was that an off-flavor was detected. From these results, it is suggested that the fatty acid generated was changed depending on the substrate specificity of the enzyme, so that the perception of off-flavor was thereby influenced.

### [Example 2]

95 mL of coffee was dispensed into a reaction vessel. A lipase derived from *Candida cylindracea* was added to the coffee to 0.1 %W/V (503 U lipase activity per gram of the coffee). The reaction was carried out in a 30°C water bath for 50 minutes. After completion of the reaction, the enzyme was inactivated by being treated in a boiling water bath for 10 minutes, and a sample for acid value measurement was then collected. The processed coffee obtained after the reaction was transferred to a 100 mL glass vial, and stored under refrigeration at 5°C, and the presence or absence of generation of oil rings (aggregates of coffee oil) was observed. After storing for 1 week, the processed coffee was shaken, the storage at 5°C was then continued, and thereafter, the oil rings were observed.

### [Comparative Example 4]

Coffee (control) was observed in the same manner as that of Example 2, with the exception that a lipase derived from *Candida cylindracea* was not added, after the coffee was produced in the same manner as that of Example 1.

### [Evaluation 1] Oil ring formation

The coffee or the processed coffee obtained from Example 2 and Comparative Example 4 was stored at 5°C, and the presence or absence of oil ring formation was observed on Days 0, 7, 14, 21, 28, 35, 42, and 72. The results were evaluated using the following scale: "+++: Thick oil ring formed, ++: Oil ring formed, +: Slight oil ring formed, and -: No oil ring formed."

**[Table 5]**

| Observation of oil rings in processed coffee | | | | | | | | | |
|---|---|---|---|---|---|---|---|---|---|
| | | Store in refrigerator at 5°C | | | | | | | |
| | | Day 0 | Day 7 | Day 14 | Day 21 | Day 28 | Day 35 | Day 42 | Day 72 |
| Comp. Ex. 4 | Untreated | +++ | +++ | +++ | +++ | +++ | +++ | +++ | +++ |
| | with enzyme | | | | | | | | |
| Example 2 | Lipase derived from *Candida cylindracea* | - | - | - | - | + | + | + | + |

As shown in the table above, it was demonstrated that, by incorporating an industrially feasible step (such as shaking during transportation), the period during which no oil rings are formed at all can be extended from 21 days of the storage to 28 days or more of storage. In addition, it was also demonstrated that a long-term storage (72 days) also becomes possible.

### [Example 3]

95 mL of the coffee liquid shown in Table 1 was dispensed into a reaction vessel. A lipase derived from *Candida cylindracea* was added to the coffee to 0.05 %W/V (252 U lipase activity per gram of the coffee). The reaction was carried out in a 10°C constant temperature bath for precisely 1 hour. After completion of the reaction, the enzyme was inactivated by being treated in a boiling water bath for 10 minutes. The processed coffee obtained after the reaction was transferred to a 100 mL glass vial, and stored under refrigeration at 5°C, and the presence or absence of generation of oil rings (aggregates of coffee oil) was observed.

### [Comparative Example 5]

Coffee (control) was observed in the same manner as that of Example 3, using the coffee liquid shown in Table 1, with the exceptions that a lipase derived from *Candida cylindracea* was not added.

### [Evaluation 1] Oil ring formation

The coffee or the processed coffee obtained from Example 3 and Comparative Example 5 was stored at 5°C, and the presence or absence of oil ring formation was observed on Day 28. The results were evaluated using the following scale: "+++: Thick oil ring formed, ++: Oil ring formed, +: Slight oil ring formed, and -: No oil ring formed."

**[Table 6]**

| Observation of oil rings in processed coffee | | |
|---|---|---|
| | | Day 28 |
| Comp. Ex. 5 | Untreated with enzyme | +++ |
| Example 3 | Lipase derived from *Candida cylindracea* | + |

It was revealed that, even in the case of performing a lipase treatment at 10°C, it has a certain effect of reducing oil ring formation.

## Claims

1. An enzyme agent containing a lipase derived from Candida sp., which is for suppressing aggregation of coffee oil.

2. A processed coffee, in which the enzyme agent according to claim 1 is allowed to act on coffee.

3. The processed coffee according to claim 2, in which the moisture content of the coffee is 80% by mass or more.

4. A method for producing a processed coffee, including allowing a lipase derived from Candida sp. to act on coffee.

5. The method for producing a processed coffee according to claim 4, in which the moisture content of the coffee is 80% by mass or more.

6. A method for suppressing aggregation of coffee oil, including allowing a lipase derived from Candida sp. to act on coffee.
